# EUROPEAN PATENT SPECIFICATION

(11) **EP 1 593 364 B1**
(45) Date of publication and mention of the grant of the patent: **15.10.2008**
(21) Application number: 04010624.7
(22) Date of filing: 05.05.2004
(51) Int. Cl.: A61Q 5/00, A61K 8/03, A61K 8/92, A61K 8/34, A61Q 17/04

(54) **Three-phase hair care composition**
Dreiphasen-Haarpflegemittel
Composition en trois phases pour soin des cheveux

(43) Date of publication of application: 09.11.2005
(73) Proprietor: KPSS-Kao Professional Salon Services GmbH, 64297 Darmstadt (DE)
(72) Inventor: Schupp, Bettina, 64404 Bickenbach (DE); Bistram, Vera, 64683 Einhausen (DE)

(56) References cited:
- EP-A- 0 882 442
- GB-A- 2 259 015
- US-A1- 2003 048 693
- US-A1- 2003 161 852
- US-B1- 6 346 507
- PATENT ABSTRACTS OF JAPAN vol. 0171, no. 02 (C-1031), 2 March 1993 (1993-03-02) & JP 4 290810 A (KUROODA JIYAPAN KK), 15 October 1992 (1992-10-15) & DATABASE CA [Online] CHEMICAL ABSTRACTS SERVICE, COLUMBUS, OHIO, US; TSUJI, TADASHI ET AL: "Three layer-type bases and cosmetic compositions containing monofatty acid polyoxyethylene glycerins as surfactants" retrieved from STN Database accession no. 1993:66618 & JP 04 290810 A2 (KURODA JAPAN K. K., JAPAN) 15 October 1992 (1992-10-15)

## Description

This invention relates to a cosmetic composition comprising optically separated three liquid phases, which mixes homogenously upon shaking and separates again into three phases in a short period of time. The composition shows excellent caring performance and improves manageability and shine of keratin fibres, especially of hair.

Hair care products have been known for many years in various product forms either in appearance or in application. Emulsion type of rinse off composition had been goveming the market till recent availability of leave in compositions. Various types of leave-in products are on the market. Their caring effect varies depending on their content, application and type of the keratin fibres treated with. Although the state of the art is well developed, there is still need for further development.

Two-phase cosmetic composition is disclosed for skin for example in EP 494 391. As well, a two-phase composition is disclosed in DE 42 15 502. Such kinds of compositions are very much preferred by the end users because of their attractive appearance especially when the two-separated phase are coloured differently.

US 2003/0003069 A1 discloses multiphase cleansing products comprising oil, surfactant and aqueous phases. DE 42 27 779 A1 discloses three-phase skin care compositions.

EP 882 442 A1 discloses three phase cosmetic compositions comprising mineral oil, glycerin and PEG-7 glyceryl cocoate as skin care agent. The documents does not mention anything on UV filters.

US 2003/048693 A1 is on transparent microemulsion comprising mineral oil, glycerin and PEG-7 glyceryl cocoate. The document does not mention transparent three phase composition and UV filter content of such.

US 6,346,507 discloses liquid crystal compositions which may comprise components of claim 1 except UV filter.

JP 4-290810 (abstract) discloses compositions containing mono fatty acid polyoxyethylene glycerins as surfactants such as PEG-7 gylceryl cocoate, mineral oil and glycerine. The document does not mehtion UV filters.

GB 2 259 015 A disposes three-phase cosmetic compositions with an oii phase, silicone and water. The compositions may furthermore comprise UV filters. The document does not teach compositions with ethoxylated fatty acid glyceryl ester and polyol.

None of the above mentioned documents disclose a composition claimed with the present invention for hair care in order to provide hair with improved manageability and less fly-aways.

Inventors of the present invention have surprisingly found out that composition comprising an oil, preferably a mineral oil, ethoxylated glyceryl fatty acid mono ester, a polyol and at least one UV filter separates into three phases in a short period of time and gives excellent caring properties to keratin fibres and especially makes manageability of hair excellently easy as well as prevents any fly-aways on hair surface.

Thus, it is an object of the present invention to develop a composition separated into three liquid phases, which mixes homogenously upon shaking and separates again into three phases in a short period of time when shear is removed.

It is further an object of the present invention that composition shows excellent caring performance on keratin fibres especially hair and makes manageability of hair excellently easy in addition to preventing fly-aways on the hair surface. The term hair surface here refers to a surface presented by a bundle of hair and explicitly not to a surface of single hair fibre.

Composition of the present invention comprises a liquid oil, preferably mineral oil, an ethoxylated glyceryl fatty acid mono ester, a polyol and at least one UV filter. The concentrations of the individual components do not play a role in separation of the composition into three phases. In other words, the ratio of the three phases can be chosen freely without effecting the separation into three phases.

Preferably composition of the present invention comprises oily phase at a concentration of 10 to 60% by weight, the second phase being an ethoxylated glyceryl fatty acid mono at a concentration of 10 to 60% by weigh and the third phase being a polyol 10 to 60% by weight, calculated to total composition.

Preferred liquid oil is mineral oil as the oily phase which forms the upper phase. In the detailed experimental study, it is found out that density of the mineral oil seems to be important in separation of the compositions into three phases, especially when clear appearance is preferred. Although principally it is possible to use any mineral oil, the preferred mineral oils are those with density values ranging from 0.83 to 0.89 g/cm³ and more preferably from 0.83 to 0.87 g/cm³ and most preferably from 0.835 to 0.86 g/cm³.

Into the upper oily phase it is possible to add additional oil substances selected from silicone oils either volatile or non-volatile, natural and synthetic oils. Among silicone oils those can be added to the compositions include dimethicone, dimethiconol, polydimethylsiloxane, DC fluid ranges from Dow Corning. Natural oils suitable are such as olive oil, almond oil, avocado oil, weizenkeim oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil. Synthetic oily compounds such as fatty acid esters are as well suitable for the composition of the present invention. Those are such as isopropyl myristate, palmitate, stearate and isostearate, oleyl oleate, isocetyl stearate, hexyl laurate, dibutyl adipate, dioctyl adipate, myristyl myristate and oleyl erucate.

Additionally, all oil soluble compounds can be added into the oil phase of the compositions. Oil soluble UV flirters are specially preferred compounds to be added into the oil phase for protecting hair from damaging effects of UV rays.

The second phase of the compositions of the present invention comprises ethoxylated glyceryl fatty acid mono ester. The most preferred is the PEG-7 glyceryl cocoate which is known with its trade name Cetiol HE from Cognis.

The third phase is the most hydrophilic phase comprising polyols. Most preferred polyol is glycerin.

In principal all water-soluble compounds can be added into the third phase. One of the compounds is water soluble UV filters, similar to the oil soluble ones for protecting hair from damaging effects of UV rays.

The UV absorbers given below which are used for the purposes of the present invention are not of course intended to be limiting. Among the examples, as a rule the salts are water soluble and acids are oil soluble ones. Certainly there are compounds may be soluble in both medium as th purpose of the invention is definitively not identifying the exact phase where the additional compounds, such as UV filters, are found. Certainly partitioning is not excluded of the compounds between the three phases. The examples are: 4-aminobenzoic acid, ethyl 4-aminobenzoate, 2-ethylhexyl 4-dimethylaminobenzoate, glycerol 4-aminobenzoate, homomenthyl salicylate (homosalate), 2-ethylhexyl salicylate, triethanolamine salicylate, 4-isopropylbenzyl salicylate, menthyl anthranilate, ethyl diisopropylcinnamate, 2-ethylhexyl p-methoxycinnamate, methyl diisopropylcinnamate, isoamyl p-methoxycinnamate, p-methoxycinnamic acid diethanolamine salt, isopropyhl p-methoxycinnamate, 2-ethylhexyl 2-cyano- 3,3-diphenylacrylate, ethyl 2-cyano-3,3'-diphenylacrylate, 2- phenylbenzimidazole-5-sulphonic acid and salts thereof, 3-(4'- trimethylammonium)-benzylidene-bornan-2-one methylsulphate, terephthalylidene-dibomanesulphonic acid and salts, 4-t-butyl-4'- methoxydibenzoylmethane, β-imidazole-4(5)-acrylic acid (urocaninic acid), 2-hydroxy-4-methoxybenzophenone, 2-hydroxy-4-methoxybenzophenone-5- sulphonic acid, dihydroxy-4-methoxybenzophenone, 2,4- dihydroxybenzophenone, tetrahydroxybenzophenone, 2,2'-dihydroxy-4,4'- dimethoxybenzophenone, 2-hydroxy-4-n-octoxybenzophenone, 2-hydroxy-4- methoxy-4'-methylbenzophenone, 3-(4'-sulpho)benzylidene-bornan-2-one and salts thereof, 3-(4'-methylbenzylidene)camphor, 3-benzylidenecamphor, 3, 3'-(1,4-phenylenedimethine)-bis-(7,7-dimethyl-2-oxo-bicyclo-[2.2.1]heptane-1-methanesulphonic acid and salts thereof, 4- isopropyidibenzoylmethane, 2,4,6-trianilino-(p-carbo-2'-ethylhexyl-1 '- oxy)-1,3,5-triazine, phenylene-1,4-bis-(2-benzimidazyl)-3,3'-5,5'- tetrasulphonic acid and salts thereof, particularly the corresponding sodium, potassium or triethanolammonium salts, in particular the disodium salt, 2,2'-(1,4-phenylene)-bis-(1 H-benzimidazole-4,6-disulphonic acid), monosodium salt, N-[(2 and 4)-[2-(oxoborn-3-ylidene)methyl]benzyl] acrylamide polymer, phenol, 2-(2H-benzotriazol-2-yl)-4-methyl-6-(2-methyl- 3-(1,3,3,3-tetramethyl-1-(trimethylsilyl)-oxy)-disiloxyanyl)-propyl, 4,4'- [(6-[4-(1,1-dimethyl)aminocarbonyl)-phenylamino]-1,3,5-triazine-2,4-diyl) diimino]-bis-(benzoate-2-ethylhexylester), 2,2'-methylene-bis-(6-(2H- benzotriazol-2-yl)-4-1,1,3,3-tetramethylbutyl)-phenol), 2,4-bis-[4-(2- ethylhexyloxy)-2-hydroxyphenyl]-1,3,5-triazine, benzylidenemalonate- polysiloxane, glyceryl ethyl hexanoate dimethoxycinnamate, disodium 2,2'- dihydroxy-4,4'-dimethoxy-5,5'-disulphobenzophenone, dipropylene glycol salicylate, sodium hydroxy-methoxybenzophenone-sulphonate, tris(2- ethylhexyl) 4,4',4-(1,3,5-triazine-2,4,6-triyltriimino)-tris-benzoate, 2, 4-bis-[{(4-(2-ethyl-hexyloxy)-2-hydroxy}-phenyl]-6-(4-methoxyphenyl)-1,3, 5-triazine, 2,4-bis-[{(4-(3-sulphonato)-2-hydroxy-propyloxy)-2-hydroxy}- phenyl]-6-(4-methoxyphenyl)-1,3,5-triazine sodium salt, 2,4-bis[{(3-(2-propyloxy)-2-hydroxy-propyloxy)-2-hydroxy}-phenyl]-6-(4-methoxy-phenyl)-1, 3,5-triazine, 2,4-bis-[{4-(2-ethyl-hexyloxy)-2-hydroxy}-phenyl]-6-[4-(2- methoxyethyl-carbonyl)-phenylamino]-1,3,5-triazine, 2,4-bis-[{4-(3-(2- propyloxy)-2-hydroxy-propyloxy)-2-hydroxy}-phenyl]-6-[4-(2-ethylcarboxyl)- phenylamino]-1,3,5-triazine, 2,4-bis-[{4-(2-ethyl-hexyloxy)-2-hydroxy}- phenyl]-6-(1-methyl-pyrrol-2-yl-)-1,3,5-triazine, 2,4-bis-[{4-tris-(trimethylsiloxy-silylpropyloxy)-2-hydroxy}-phenyl]-6-(4-methoxyphenyl)-1, 3,5-triazine, 2,4-bis-[{4-(2"-methylpropenyloxy)-2-hydroxy}-phenyl]-6-(4-methoxyphenyl)-1,3,5-triazine, 2,4-bis-[{4-(1',1',1',5',5',5'-hepta- methylsiloxy-2"-methyl-propyloxy)-2-hydroxy}-phenyl]-6-(4-methoxyphenyl)- 1,3,5-triazine. Concentration of UV filters, either water or oil soluble can vary between 0.05 to 1%, preferably 0.05 to 0.75%, and most preferably 0.05 to 0.5% by weight calculated to total composition.

Additionally, other polyols such as panthenol in admixture with glycerin may be used as well.

Further additionally, especially to the polyol phase, polyethyleneglycoles can be added, which are known with trade names Carbowax PEG from Union Carbide and Polyox WSR range from Amerchol, polyglycerin, polyethyleneglycol mono or di fatty acid esters having general formula,

R₁ CO (O CH₂ CH₂)ₙ OH

R₁ CO (O CH₂ CH₂)ₙ O OC R₂

where R₁ and R₂ are independent from each other saturated, unsaturated or branched or non-branched alkyl chain with 7 to 21 C atoms and n is typically 2 - 100. Typical concentration range for any of the additional conditioners mentioned above other than cationic conditioning compounds can be 0.01 - 5% by weight, preferably 0.05 - 3%, more preferably 0.05 - 1 % by weight calculated to the total composition.

The compositions according to the invention can also comprise further agents, such as protein hydrolyzates and polypeptides, e.g. keratin hydrolyzates, collagen hydrolyzates of the type "Nutrilan®" or elastin hydrolyzates, as well as, in particular vegetable, optionally cationized protein hydrolyzates, for example "Gluadin®".

Additional natural plant extracts can as well form part of the compositions of the present invention. Those are incorporated usually in an amount of about 0.01 % to about 10 %, preferably 0.05 % to 7.5 %, in particular 0.1 % to 5 % by weight, calculated to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon®" products, "Herbasol^{R} ", "Sedaplant^{R}" and "Hexaplant^{R}". Extracts and the preparation thereof are also described in "Hagers Handbuch der pharmazeutischen Praxis", 4^{th} Ed..

Among the natural ingredients in the form of an extract, especially preferred component of the composition according to the invention is green tea extract. This tea extract is obtained from the leaves, leaf buds and tender stems of the tea shrub, Camellia sinensis or Camellia oleifera, by aqueous or hydro-alcoholic extraction and subsequent spray-drying. In difference to black tea, green tea is a non-fermented product obtained from the Thea sinensis or Thea assamica species. An overview of the biological and pharmacological effects of green tea and the ingredients thereof can be found, e.g., in an article by A. Pistorius, "Seifen-Öle-Fette-Wachse-Journal", Volume 122., No. 7/1996, pages 468 to 471, to which reference is made. The content of green tea extract is variable in the compositions according to the invention. It preferably ranges from 0.01 % to 10 %, preferably 0.05 % to 5% by weight, calculated to the total composition.

The compositions of the present invention are not water free compositions. However, high water content may cause stability problems especially the composition may turn into two phase product, as well as especially in the presence of additional surface active compounds, may turn into an emulsion. Therefore the water content of the compositions should not exceed 20% by weight calculated to the total composition.

Three-phase cosmetic composition according to the present invention may comprise additional optional ingredients. In the selection of the optional ingredients care should be given to primarily phase separation as well to the appearance of a composition so obtained. In certain cases, below will be mentioned specifically for each optional compound, the volumes of the phases after complete separation may change, though the composition is still a three-phase composition with the same appearance. In other cases, appearance may be influenced such as from transparent to turbid appearance etc.

Optionally, the composition of the present invention can comprise one or more conditioning ingredients selected from cationic polymers and cationic amphiphilic ingredients, and their mixtures. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride.

Furthermore, chitosan and chitin can also be included in the compositions as cationic natural polymers.

Furthermore, it has especially been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

The cationic polymers also include the quaternized products of graft polymers from organopolysiloxanes and polyethyl oxazolines described in EP-A 524 612 and EP-A 640 643.

Cationic amphiphilic ingredients as conditioning agents according to the present invention are represented with the general formula below: 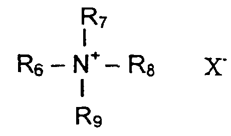 where R₆ is a saturated or unsaturated, branched or non-branched alkyl chain with 8-22 C atoms or

R₁₀ CO NH (CH₂)ₙ

where R₁₀ is saturated or unsaturated, branched or non-branched alkyl chain with 7-21 C atoms and n has typical value of 0 - 4 or

R₁₁ CO O (CH₂)ₙ

where R₁₁ is saturated or unsaturated, branched or non-branched alkyl chain with 7-21 C atoms and n has typical value of 0 - 4, and
R₇ is a hydrogen, lower alkyl chain with 1 to 4 carbon atoms, saturated or unsaturated, branched or non-branched alkyl chain with 8-22 C atoms or

R₁₀ CO NH (CH₂)ₙ

where R₁₀ is saturated or unsaturated, branched or non-branched alkyl chain with 7-21 C atoms and n has typical value of 0 - 4 or

R₁₁ CO O (CH₂)ₙ

where R₁₁ is saturated or unsaturated, branched or non-branched alkyl chain with 7-21 C atoms and n has typical value of 0 - 4, and
R₈ and R₉ are independent from each other H or lower alkyl chain with 1 to 4 carbon atoms, and X is chloride, bromide or methosulfate.

Suitable cationic amphiphilic compounds as conditioning agents are, for example, long-chain quaternary ammonium compounds which can be used alone or in admixture with one another, such as cetyl trimethyl ammonium chloride, myristoyl trimethyl ammonium chloride, trimethyl cetyl ammonium bromide, stearyl trimethyl ammonium chloride, dimethyl stearyl ammonium chloride, dimethyl dihydrogenated tallow ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate.

From the above quaternary ammonium compounds disclosed with the general formula, especially preferred are those compounds known per se and are on the market, for example, under the trade names "Schercoquat®", "Dehyquart® F30" and "Tetranyl®". Use of these compounds, the so-called "esterquats", in hair care compositions is described, for example, in WO-A 93/107 48, WO-A 92/068 99 and WO-A 94/166 77, wherein, however, there is no reference made to the combinations according to the present invention and the advantageous properties thereof.

Again from the above quaternary ammonium compounds disclosed with the general formula, especially preferred are these compounds are known **per se** and on the market, for example, under the trade name "INCROQUAT® HO" or "OCS". Those compounds are known with a general ingredient category under "amidoquat" in the cosmetic industry.

The cationic conditioners can be present in the compositions at a concentration of 0.01 to 2% preferably up to 1% by weight calculated to total composition.

Additionally, compositions of the present invention may comprise other conventional cosmetic ingredients such as preservatives, fragrances, sequestering agents, dyestuffs and vitamins.

Appearance of the three phase compositions of the present invention can be variable, either clear or turbid, colored and colorless can be listed as the options. Those can certainly be the properties of one and/or two and/or three phases. For example, one phase can be turbid, opaque, the other phase can be clear and colored and the other phase can be clear and colorless. It can as well be, bottom phase clear and colorless (glycerin phase) and middle phase clear and colored (ethoxylated glyceryl fatty acid mono ester phase) and upper phase (mineral oil phase) clear and colorless. Those are only non-limiting examples among many possibilities.

For introducing turbidity, especially to the glycerin phase - the most hydrophilic phase, styrene/vinylpyrrolidone copolymer is preferred. It is available as 40% aqueous dispersion under the trade name Antara 430 from ISP Corporation.

The composition of the present invention can be used on wet and dry hair. On wet hair, it is applied uniformly onto shampooed and towel dried hair after shaking the composition until homogeneity and combed through and dried. On dry hair, it can be distributed streak wise and comb through.

For any composition of the present invention, the period needed for a clear separation into 3 phases is determined by its content. A simple composition comprising only 3 ingredients being, mineral oil, PEG-7 gyleceryl cocate and glycerin, the period is up to 10 min. When additional ingredients are added into any of the phases the time required for clear separation into 3 phases is extended.

Following are non-limiting examples to demonstrate the invention

### Example 1

| Mineral oil (Paraffinum liquidum) | 32 % by weight |
|---|---|
| PEG-7 Glyceryl cocoate | 32 |
| Glycerin | 32 |
| Ethylhexyl methoxycinnamate | 0.3 |
| Fragrance | 0.7 |
| Green-tea extract | 3.0 |

Composition is prepared by combining ethylhexyl methoxycinnamate and fragrance with mineral oil and other ingredients are mixed into this until homogeneity. Preparation is filled into a clear bottle and after 10 min separated into three clear colorless phases. From Green tea extract slight coloration is observed in middle and lower phases.

This composition is tested in a half side comparison test against a two-phase product well known in the hair cosmetic market consisting of silicone oil around 25% and water phase comprising polymers and minor amount of hair care ingredients. The test was carried out with 10 female volunteers having shoulder length, damaged hair and having problem with manageability, especially with fly-aways, of their hair. Hair was shampooed uniformly with a commercially available shampoo and 1.5 g of the above composition and equal amount of the two-phase composition was applied onto wet hair on each side and both sides were dried with an air drier. Volunteer's hair was then evaluated by hair dressers.

In wet stage, the most obvious preference was in the combing of the side treated with the composition of example 1. The preference was 70%, in the remaining 30% of the cases there was no clear preference on either side.

In the dry stage, in 70% of the cases the side treated with the composition according to example 1 is preferred for better combability. For fly-aways in all cases (100%) the preference was on the side of the example 1. Shine was evaluated to be as well better in all cases.

Similar results are obtained with the following compositions.

### Example 2

| Mineral oil (Paraffinum liquidum) | 32 % by weight |
|---|---|
| PEG-7 Glyceryl cocoate | 32 |
| Glycerin | 32 |
| Ethylhexyl methoxycinnamate | 0.3 |
| Polyquaternium-11 * | 1.0 |
| Fragrance | 0.7 |
| Styrene/vinylpyrrolidone copolymer** | 1.0 |
| Panthenol 50% | 1.0 |

| | |
|---|---|
| *: Gafquat 440 **: Antara 430 | |

The composition is prepared in the same way as the example 1 and filled into a bottle. Within 15 min it is separated into 3 phases , the upper two are being clear and the bottom phase is turbid - white.

### Example 3

| Mineral oil (Paraffinum liquidum) | 29 % by weight |
|---|---|
| PEG-7 Glyceryl cocoate | 30 |
| Glycerin | 40 |
| Ethylhexyl methoxycinnamate | 0.3 |
| Fragrance | 0.7 |
| Chlorophyllin - Copper | q.s (for achieving coloured appearance) |

The composition is prepared in the same way as the example 1 and filled into a bottle. Within 10 min it is separated into 3 phases , all phases are clear and the middle phase is colored green.

### Example 4

| Mineral oil (Paraffinum liquidum) | 15 % by weight |
|---|---|
| PEG-7 Glyceryl cocoate | 24 |
| Glycerin | 60 |
| Ethylhexyl methoxycinnamate | 0.3 |
| Fragrance | 0.7 |

### Example 5

| Mineral oil (Paraffinum liquidum) | 50 % by weight |
|---|---|
| PEG-7 Glyceryl cocoate | 30 |
| Glycerin | 19 |
| Ethylhexyl methoxycinnamate | 0.3 |
| Fragrance | 0.7 |

### Example 6

| Mineral oil (Paraffinum liquidum) | 20 % by weight |
|---|---|
| Dimethicone | 5 |
| PEG-7 Glyceryl cocoate | 30 |
| Glycerin | 44 |
| Ethylhexyl methoxycinnamate | 0.3 |
| Fragrance | 0.7 |

## Claims

1. Cosmetic composition for keratin fibers especially for hair consisting of optically separated three phases comprising an oily fist phase, an ethoxylated gylceryl fatty acid monoester comprising second phase and a third phase comprising polyol and comprising at least one UV filter.

2. Cosmetic composition according to claim 1 **characterized in that** oily phase comprises mineral oil.

3. Cosmetic composition according to claim 1 **characterized in that** second phase comprises PEG-7 glyceryl cocoate.

4. Cosmetic composition according to claim 1 **characterized in that** third phase comprises glycerin.

5. Cosmetic composition according to any of the preceding claims **characterized in that** it comprises each phase at a concentration of 10 to 60% by weight calculated to total composition.

6. Cosmetic composition according to any of the preceding claims **characterized in that** the mineral oil has a density between 0.83 and 0.89 g/cm³.

7. Cosmetic composition according to any of the preceding claims **characterized in that** it comprises additionally hair conditioning one or more cationic polymer and/or cationic amphiphilic ingredient.

8. Cosmetic composition according to any of the preceding claims **characterized in that** one and/or two and/or three phases are either clear and colorless or clear and colored or turbid and colored.

9. Use of composition according to any of the claims 1 to 8 on wet and dry hair for improving shine and manageability of hair.

## Patentansprüche

1. Kosmetische Zusammensetzung für Keratinfasern, besonders für Haare die aus drei optisch getrennten Phasen besteht, einer ersten öligen Phase, einer ethoylierten Glycerylfettsäuremonoester enthaltenden zweiten Phase und einer Polyol enthaltenden dritten Phase, und die mindestens einen UV- Filter enthält.

2. Kosmetische Zusammensetzung nach Anspruch 1, **dadurch gekennzeichnet, dass** die ölige Phase Mineralöl enthält.

3. Kosmetische Zusammensetzung nach Anspruch 1, **dadurch gekennzeichnet, dass** die zweite Phase PEG-7 Glycerol Cocoate enthält.

4. Kosmetische Zusammensetzung nach Anspruch 1, **dadurch gekennzeichnet, dass** die dritte Phase Glycerin enthält.

5. Kosmetische Zusammensetzung nach einem der vorhergehenden Ansprüche, **dadurch gekennzeichnet, dass** jede Phase in einer Menge von 10 bis 60 Gew.- %, berechnet auf die Gesamtzusammensetzung, entfalten ist.

6. Kosmetische Zusammensetzung nach einem der vorhergehenden Ansprüche, **dadurch gekennzeichnet, dass** das Mineralöl eine Dichte zwischen 0,83 und 0,89 g/cm³ hat.

7. Kosmetische Zusammensetzung nach einem der vorhergehenden Ansprüche, **dadurch gekennzeichnet, dass** sie als zusätzliche Haarkonditionierung ein oder mehrere kationische Polymere und/oder kationische amphiphilische Bestandteile enthält.

8. Kosmetische Zusammensetzung nach einem der vorhergehenden Ansprüche, **dadurch gekennzeichnet, dass** eine und/oder zwei und/oder drei Phasen entweder klar und farblos, oder klar und gefärbt, oder trübe und gefärbt sind.

9. Verwendung der Zusammensetzung nach einem der Ansprüche 1 bis 8 auf feuchtem und trockenem Haar, zur Verbesserung der Leuchtkraft und der Bearbeitbarkeit der Haare.

## Revendications

1. Composition cosmétique pour fibres de kératine, en particulier pour les cheveux, constituée de trois phases optiquement séparées comprenant une première phase huileuse, une deuxième phase comprenant un monoester d'acide gras glycérylique éthoxylé et une troisième phase comprenant un polyol, et comprenant au moins un filtre UV.

2. Composition cosmétique selon la revendication 1, **caractérisée en ce que** la phase huileuse comprend une huile minérale.

3. Composition cosmétique selon la revendication 1, **caractérisée en ce que** la deuxième phase comprend du cocoate glycérylique de PEG-7.

4. Composition cosmétique selon la revendication 1, **caractérisée en ce que** la troisième phase comprend de la glycérine.

5. Composition cosmétique selon l'une quelconque des revendications précédentes, **caractérisée en ce qu'**elle comprend chaque phase à une concentration de 10 à 60 % en poids, calculée par rapport à la composition totale.

6. Composition cosmétique selon l'une quelconque des revendications précédentes, **caractérisée en ce que** l'huile minérale a une densité comprise entre 0,83 et 0,89 g/cm³.

7. Composition cosmétique selon l'une quelconque des revendications précédentes, **caractérisée en ce qu'**elle comprend en outre un ou plusieurs ingrédients polymères cationiques et/ou amphiphiles cationiques pour le conditionnement des cheveux.

8. Composition cosmétique selon l'une quelconque des revendications précédentes, **caractérisée en ce qu'**une et/ou deux et/ou les trois phases sont limpides et incolores ou limpides et colorées ou troubles et colorées.

9. Utilisation d'une composition selon l'une quelconque des revendications 1 à 8 sur des cheveux humides et secs pour améliorer la brillance et l'aptitude à être coiffés des cheveux.
